# EUROPEAN PATENT SPECIFICATION

(11) **EP 3 970 735 B1**
(45) Date of publication and mention of the grant of the patent: **12.03.2025**
(21) Application number: 20810761.5
(22) Date of filing: 18.05.2020
(51) Int. Cl.: A61K 38/12, A61K 38/05, A61K 38/29, A61P 19/00, A61P 19/10, A23L 33/18

(54) **USE OF COMPOSITION FOR PREVENTING, AMELIORATING, OR TREATING BONE LOSS DISORDERS, COMPRISING CYCLO-HISPRO (CHP) AND PARATHYROID HORMONE**
VERWENDUNG EINER ZUSAMMENSETZUNG ZUR VORBEUGUNG, VERBESSERUNG ODER BEHANDLUNG VON KNOCHENSCHWUND MIT CYCLO-HISPRO (CHP) UND PARATHYROIDHORMON
UTILISATION D'UNE COMPOSITION POUR LA PRÉVENTION, L'AMÉLIORATION OU LE TRAITEMENT DE TROUBLES DE LA PERTE OSSEUSE, COMPRENANT LE CYCLO-HISPRO (CHP) ET L'HORMONE PARATHYROÏDE

(30) Priority: 17.05.2019 KR 20190058369
(43) Date of publication of application: 23.03.2022
(73) Proprietor: Novmetapharma Co., Ltd., Seoul 06050 (KR)
(72) Inventor: JUNG, Hoe Yune, Pohang-si, Gyeongsangbuk-do 37668 (KR); LEE, Do Hyun, Pohang-si, Gyeongsangbuk-do 37669 (KR); LEE, Heon Jong, Incheon 21048 (KR)
(74) Representative: Maiwald GmbH
(86) International application number: PCT/KR2020/006491
(87) International publication number: WO 2020/235900

(56) References cited:
- WO-A1-2007/066841
- WO-A2-2011/093599
- WO-A2-2013/015611
- KR-A- 20060 105 735
- KR-A- 20090 116 836
- US-A1- 2009 004 291
- DOUGLAS A HORTON ET AL: "Exploring privileged structures: the combinatorial synthesis of cyclic peptides", JOURNAL OF COMPUTER-AIDED MOLECULAR DESIGN, KLUWER ACADEMIC PUBLISHERS, DO, vol. 16, no. 5-6, 1 May 2002 (2002-05-01), pages 415 - 431, XP019248059, ISSN: 1573-4951
- ALAN D. BORTHWICK: "2,5-Diketopiperazines: Synthesis, Reactions, Medicinal Chemistry, and Bioactive Natural Products", CHEMICAL REVIEWS, vol. 112, no. 7, 11 July 2012 (2012-07-11), US, pages 3641 - 3716, XP055347546, ISSN: 0009-2665, DOI: 10.1021/cr200398y
- ADRIáN SáNCHEZ ET AL: "Bioactive peptides: A review", FOOD QUALITY AND SAFETY, vol. 1, no. 1, 1 March 2017 (2017-03-01), pages 29 - 46, XP055559383, ISSN: 2399-1399, DOI: 10.1093/fqs/fyx006
- LEE HYUN JUNG ET AL: "Preparation of Yeast Hydrolysate Enriched in Cyclo-His-Pro (CHP) by Enzymatic Hydrolysis and Evaluation of Its Functionality", JOURNAL OF FOOD SCIENCE AND NUTRITION, vol. 20, no. 4, 31 December 2015 (2015-12-31), pages 284 - 291, XP093052575, ISSN: 2287-1098, Retrieved from the Internet <URL:http://dx.doi.org/10.3746/pnf.2015.20.4.284> DOI: 10.3746/pnf.2015.20.4.284
- ROSENTHAL, M. J.: "Effects of arachidonic acid and cyclo (his-pro) on zinc transport across small intestine and muscle tissues", LIFE SCIENCES, vol. 70, no. 3, December 2001 (2001-12-01), pages 337 - 348, XP055762882
- ATIK, O. S.: "Etiology of senile osteoporosis: a hypothesis", CLINICAL ORT HOPAEDICS AND RELATED RESEARCH, vol. 443, February 2006 (2006-02-01), pages 25 - 27, XP055762883

## Description

### [Technical Field]

The present invention relates to a use of a composition including cyclo(hispro) (CHP) and parathyroid hormone (PTH) for preventing, improving or treating a bone loss-related disease.

### [Background Art]

Bone modeling and remodeling play an important role in osteogenesis, and bone growth and metabolism. Bone modeling starts from an embryonic stage and continues until adolescence and manhood, in which skeletal maturation occurs and thus its growth stops, and the maximum bone mass is formed between the twenties and early thirties. Afterward, for about 30 years, a bone remodeling process for removing bone and replenishing it is repeated, and at this time, bone formation and bone resorption work as a pair to maintain balance. After this period, bone formation cannot sufficiently catch up with bone loss induced by bone resorption, resulting in a decrease in bone mass of about 0.3 to 0.5% per year, and specifically, women have a considerable bone loss of 2 to 3% per year in early menopause.

Bone tissue constitutes the cartilage and the skeletal system, and serves as support and muscle attachment as mechanical functions, functions to protect organs and bone marrow, and conserve calcium and phosphorous ions to maintain the homeostasis thereof. Bone tissue is composed of a cell matrix such as collagen and a glycoprotein, and several types of cells such as osteoblasts, osteoclasts and osteocytes.

In addition, bone tissue is dynamic tissue that is formed by osteoblasts and repeatedly destroyed and resorbed by osteoclasts. Osteoporosis is a disease caused by an increase in bone resorption compared to bone formation due to an imbalance of osteoblasts and osteoclasts, and as the calcification of bone tissue is reduced, the density of a bone is lowered and thus the bone marrow cavity is widened, and according to the progression of symptoms, the bone becomes weaker, so it is easy to fracture even with a small impact.

For osteoporosis, various fractures, particularly, femoral fractures or vertebral fractures, easily caused by the weakening of bones, rather than the symptoms themselves restrict activities for a long time, making it impossible to have a healthy life, so it accounts for 15% of deaths of the elderly. Bone mass is affected by several factors such as genetic factors, nutritional intake, hormonal changes, differences in exercise and lifestyle, and as the cause of osteoporosis, aging, the lack of exercise, a low body weight, smoking, a low-calcium diet, menopause, and ovarian resection are known. Particularly, in women, bone loss consistently progresses after the age of 30, and dramatically progresses due to hormonal changes when women reach menopause.

As such, osteoporosis is an unavoidable symptom, although there is a difference in severity, shown in the elderly, especially postmenopausal women, and as populations are aging in developed countries, interest in osteoporosis and its therapeutic agents is gradually increasing. In addition, it is known that there is a global osteoporosis treatment-related market of about 130 billion dollars, and is expected to be bigger in the future, world-class research institutes and pharmaceutical companies are investing greatly in the development of therapeutic agents for osteoporosis, and the development of bone resorption inhibitors is being actively developed.

Recently, parathyroid hormone (PTH) has emerged as a popular therapeutic agent for osteoporosis. Unlike other therapies for reducing bone resorption, PTH increases bone mass, and further increases bone mineral density (BMD). PTH has multiple direct and indirect effects on bones. PTH increases the rate of calcium release from the bones into the blood. The chronic effect of PTH is increasing bone remodeling by increasing the number of osteocytes including osteoblasts and osteoclasts. PTH administered to osteoporotic patients significantly reduces fractures by net stimulation of bone formation, particularly, in the trabecular bone in the spine and hip joints. Bone formation is thought to occur by stimulation of osteoblasts by PTH, as osteoblasts have PTH receptors.

Under this background, the inventors have searched for a substance that can enhance the therapeutic effect on a bone loss disease when used in combination with PTH, and when PTH is used together with CHP, it was confirmed that it exhibits a synergistic effect on osteoblast differentiation and the promotion of bone formation, and thus the present invention was completed.

### [Related Art Document]

### [Patent Document]

(Patent Document 1) Korean Unexamined Patent Application No. 10-2008-0059430

### [Disclosure]

### [Technical Problem]

The present invention is directed to providing a pharmaceutical composition for preventing or treating a bone loss-related disease, which includes CHP or a pharmaceutically acceptable salt thereof; and PTH.

The present invention is also directed to providing a health functional food composition for preventing or improving a bone loss-related disease, which includes CHP or a pharmaceutically acceptable salt thereof; and PTH.

The present invention is also directed to providing a pharmaceutical composition for improving the therapeutic effect of PTH on a bone loss-related disease, which includes CHP or a pharmaceutically acceptable salt thereof.

The present invention is also directed to providing a health functional food composition for improving the effect of PTH for improving a bone loss-related disease, which includes CHP or a pharmaceutically acceptable salt thereof.

The present invention is also directed to providing a method of preventing or treating a bone loss-related disease, which includes administering an effective amount of a composition including CHP or a pharmaceutically acceptable salt thereof and PTH into a subject in need thereof.

The present invention is also directed to providing a method of improving the therapeutic effect of PTH on a bone loss-related disease, which includes administering effective amounts of CHP or a pharmaceutically acceptable salt thereof; and PTH into a subject in need thereof.

The present invention is also directed to providing a use of a composition including CHP or a pharmaceutically acceptable salt thereof and PTH for preventing or treating a bone loss-related disease.

The present invention is also directed to providing a use of a composition including CHP or a pharmaceutically acceptable salt thereof in preparation of a drug for improving the effect of PTH for preventing or treating a bone loss-related disease.

### [Technical Solution]

To solve the above-described problems, the present invention relates to a pharmaceutical composition for preventing or treating a bone loss-related disease, which includes CHP or a pharmaceutically acceptable salt thereof; and PTH.

The present invention also provides a health functional food composition for preventing or improving a bone loss-related disease, which includes CHP or a pharmaceutically acceptable salt thereof; and PTH.

The present invention also includes a pharmaceutical composition for improving the therapeutic effect of PTH on a bone loss-related disease, which includes CHP or a pharmaceutically acceptable salt thereof.

The present invention also includes a health functional food composition for improving the effect of PTH for improving a bone loss-related disease, which includes CHP or a pharmaceutically acceptable salt thereof.

The present invention also includes a method of preventing or treating a bone loss-related disease, which includes administering an effective amount of a composition including CHP or a pharmaceutically acceptable salt thereof and PTH into a subject in need thereof.

The present invention also includes a method of improving a therapeutic effect of PTH on a bone loss-related disease, which includes administering CHP or a pharmaceutically acceptable salt thereof and PTH into a subject in need thereof.

The present invention also includes a use of a composition including CHP or a pharmaceutically acceptable salt thereof and PTH in preparation of a drug for preventing or treating a bone loss-related disease.

The present invention also includes a use of a composition including CHP or a pharmaceutically acceptable salt thereof in preparation of a drug for preventing or improving the effect of PTH for preventing or treating PTH on a bone loss-related disease.

According to an exemplary embodiment of the present invention, the PTH may be PTH₁₋₃₄.

According to another exemplary embodiment of the present invention, the bone loss-related disease may be any one or more selected from the group consisting of osteoporosis, Paget's disease, alveolar bone loss, osteomalacia, and renal osteodystrophy.

According to still another exemplary embodiment of the present invention, the osteoporosis may be caused by a decrease in female hormone levels, or the destruction or inhibition of the activity of osteoblasts.

According to yet another exemplary embodiment of the present invention, the CHP or a pharmaceutically acceptable salt thereof; and PTH may be simultaneously, separately, or sequentially administered.

### [Advantageous Effects]

A composition for preventing, improving or treating a bone loss-related disease, which includes CHP and PTH according to the present invention exhibits a synergistic effect on osteoblast differentiation and promotion of bone formation and thus is effective in treatment of a bone loss-related disease. Since CHP improves PTH effects on osteoblast differentiation and the promotion of bone formation, when PTH is applied to prevention, improvement or treatment of a bone loss-related disease, CHP can be used as an adjuvant.

### [Description of Drawings]

FIGS. 1A and 1B are graphs showing an effect of increasing ALP activity by combined treatment of CHP and hPTH₁₋₃₄ in differentiation of MC3T3-E1 osteoblasts.
FIG. 2A is a graph showing an effect of increasing the expression of an ALP gene according to the combined treatment of CHP and hPTH₁₋₃₄ in differentiation of primary osteoblasts.
FIG. 2B is a graph showing an effect of increasing the expression of a BMP2 gene according to the combined treatment of CHP and hPTH₁₋₃₄ in differentiation of primary osteoblasts.
FIG. 2C is a graph showing an effect of increasing the expression of an Osteocalcin gene according to the combined treatment of CHP and hPTH₁₋₃₄ in differentiation of primary osteoblasts.
FIG. 2D is a graph showing an effect of increasing the expression of an Osterix gene according to the combined treatment of CHP and hPTH₁₋₃₄ in differentiation of primary osteoblasts.
FIG. 2E is a graph showing an effect of increasing the expression of an Runx2 gene according to the combined treatment of CHP and hPTH₁₋₃₄ in differentiation of primary osteoblasts.
FIG. 2F is a graph showing an effect of increasing the expression of a Collagen lal (Collal) gene according to the combined treatment of CHP and hPTH₁. ₃₄ in differentiation of primary osteoblasts.
FIG. 2G is a graph showing an effect of increasing the expression of an Osteoprotegerin (OPG) gene according to the combined treatment of CHP and hPTH₁₋₃₄ in differentiation of primary osteoblasts.

### [Modes of the Invention]

Hereinafter, the present invention will be described in further detail.

As described above, the inventors have searched for a substance that can enhance the therapeutic effect on a bone loss disease when used in combination with PTH, and when PTH is used together with CHP, it was confirmed that it exhibits a synergistic effect on osteoblast differentiation and the promotion of bone formation, and thus the present invention was completed.

Therefore, the present invention provides a pharmaceutical composition for preventing or treating a bone loss-related disease, which includes CHP or a pharmaceutically acceptable salt thereof; and PTH.

The present invention also provides a health functional food composition for preventing or improving a bone loss-related disease, which includes CHP or a pharmaceutically acceptable salt thereof; and PTH.

The present invention also provides a pharmaceutical composition for improving the therapeutic effect of PTH on a bone loss-related disease, which includes CHP or a pharmaceutically acceptable salt thereof; and a health functional food composition for improving the effect of PTH for improving a bone loss-related disease, which includes CHP or a pharmaceutically acceptable salt thereof.

The "cyclo-HisPro (CHP" used herein refers to a naturally-occurring circular dipeptide consisting of histidine-proline, which is a metabolite of thyrotropinreleasing hormone (TRH), or a physiologically active dipeptide which may be synthesized in the body through TRH metabolism and de novo synthesis, which is a material widely distributed throughout the brain, spinal cord and gastrointestinal tract.

Int the composition of the present invention, the CHP may be synthesized, or purchased for use. In addition, a CHP-containing material, for example, may be used after purification from a prostate extract.

The "purified" used herein indicates that CHP is more concentrated than a form obtained from nature, such as a prostate extract. Purified ingredients may be obtained through concentration from natural sources thereof, or by a chemical synthesis method.

The main components of the "prostate extract" are zinc, CHP, a prostaglandin precursor and arachidonic acid, and since it contains a high concentration of CHP, the effect of increasing BMD and a bone volume fraction, induced by CHP, and the effect of promoting osteoblast differentiation and bone formation may be reasonably predicted.

In the composition of the present invention, the "prostate extract" may be bovine or porcine prostate powder, and preferably, a form from which fat is eliminated to increase a CHP content, but the present invention is not limited thereto.

In the composition of the present invention, the "parathyroid hormone (PTH)" may be a secretory polypeptide of 84 amino acid residues with the following amino acid sequence, or a fragment thereof: Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe-Val-Ala-Leu-Gly-Ala-Pro-Leu-Ala-Pro-Arg-Asp-Ala-Gly-Ser-Gln-Arg-Pro-Arg-Lys-Lys-Glu-Asp-Asn-Val-Leu-Val-Glu-Ser-His-Glu-Lys-Ser-Leu-Gly-Glu-Ala-Asp-Lys-Ala-Asn-Val-Asp-Val-Leu-Thr-Lys-Ala-Lys-Ser-Gln (SEQ ID NO: 1).

According to one exemplary embodiment of the present invention, the PTH may be PTH₁₋₃₄ consisting of 34 amino acids at the N-terminal of a bovine or human hormone: Ser-Val-Ser-Glu-Ile-Gln-Leu-Met-His-Asn-Leu-Gly-Lys-His-Leu-Asn-Ser-Met-Glu-Arg-Val-Glu-Trp-Leu-Arg-Lys-Lys-Leu-Gln-Asp-Val-His-Asn-Phe (SEQ ID NO: 2).

PTH₍₁₋₃₄₎ also called teriparatide is currently marketed under the trade name FORTEO by Eli Lilly, Indianapolis, Indiana, for the treatment of postmenopausal women with osteoporosis at a high risk of fracture.

The term "bone loss" used herein refers to a symptom of losing bone because of an imbalance of osteoclasts and osteoblasts, and the "bone loss-related disease" includes all of diseases relating to the symptom. Accordingly, the bone loss includes all of diseases caused by a low BMD caused by losing bone due to excessively high activity of osteoclasts, or non-smooth bone formation caused by reduced activity of osteoblasts. Specific examples of the bone loss-related disease include osteoporosis, Paget's disease, alveolar bone loss, osteomalacia, and renal osteodystrophy, but the present invention is not limited thereto.

Here, the osteoporosis is one of the climacteric or menopausal symptoms, and may be caused by a decrease in female hormone levels, or the destruction or inhibition of the activity of osteoblasts.

The term "climacterium" used herein generally means a period of transition from a period of having reproductive ability to a period of disappearing reproductive ability. The climacterium is mainly used to refer to female climacterium, which corresponds to a range including all periods before and after perimenopause, as well as menopause, generally, in the age group of 40 to 60.

The composition including CHP or a salt thereof and PTH according to the present invention may prevent, improve or treat a bone loss-related disease by promoting bone formation by promotion of osteoblast differentiation.

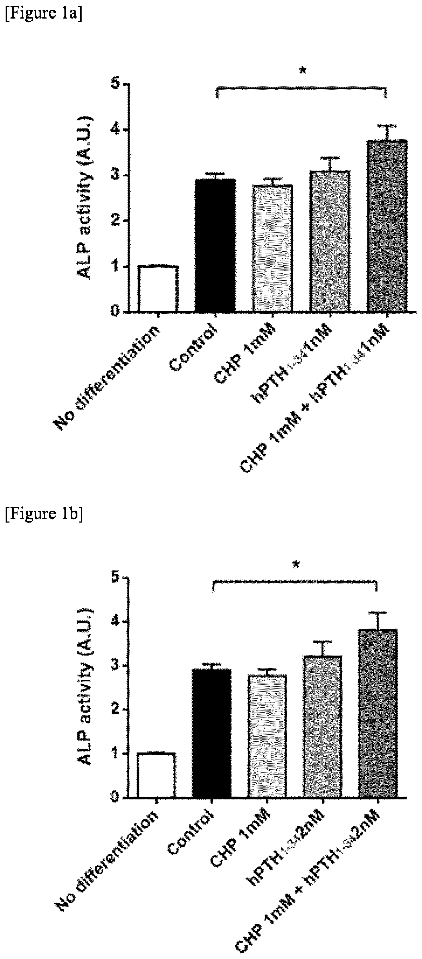

As shown in FIGS. 1A and 1B, the effect of increasing ALP activity occurring when CHP and hPTH₁₋₃₄ are administered in combination increased to a higher level than the sum of the effects of increasing ALP activity shown in single administration, confirming the synergistic effect of promoting osteoblast differentiation according to the combined administration of CHP and PTH. Alkaline phosphatase (ALP) is an early differentiation marker of osteoblasts, and when ALP activity increases, osteoblast differentiation is promoted, thereby promoting bone formation.

In the composition for preventing, improving or treating a bone loss-related disease according to the present invention, the term "prevention" refers to all actions of inhibiting or delaying the onset of a disease or symptom. In the present invention, the prevention means that the delay or inhibition of the onset of a bone loss-related disease by promoting osteoblast differentiation.

In the composition for preventing, improving or treating a bone loss-related disease according to the present invention, the term "improvement" refers to all actions involved in improving or beneficially changing a disease or its symptom, and in the present invention, means alleviation of symptoms of osteoporosis or symptoms such as alveolar bone loss, through the action of promoting osteoblast differentiation.

In the composition for preventing, improving or treating a bone loss-related disease according to the present invention, the term "treatment" refers to all actions of delaying, stopping or changing the progression of a disease or symptom, and in the present invention, means stopping, reducing, alleviating or eliminating, or changing the loss of alveolar bone or bone through an action of promoting osteoblast differentiation.

The term "synergistic effect" means that the effect generated when components are administered in combination is greater than the sum of effects generated when the components are independently administered, respectively [Chou and Talalay, Adv. Enzyme. Regul., 22:27-55, 1984]. As the CHP or a salt thereof and PTH of the present invention are administered in combination, an effect of preventing, improving or treating a bone loss-related disease is increased.

The term "administered in combination" means administration of a compound or ingredient in combination into a patient. The administration of each compound or ingredient in combination can be randomly administered at the same time or sequentially administered at different times to obtain a desired therapeutic effect.

The term "patient" refers to any individual in need of treatment, including humans, cattle, dogs, guinea pigs, rabbits, chickens, and insects. In addition, subjects include any subject participating in a clinical trial without any clinical finding of a disease, a subject participating in a clinical trial, or a subject used as a control.

The term "pharmaceutically acceptable" used herein means, when a salt is physiologically acceptable and administered to a human, it typically does not cause an allergic reaction or a similar reaction thereto, and the salt is preferably an acid addition salt formed by a pharmaceutically acceptable free acid.

The pharmaceutically acceptable salt may be an acid addition salt formed using an organic acid or inorganic acid. The organic acid may be, for example, formic acid, acetic acid, propionic acid, lactic acid, butyric acid, isobutyric acid, trifluoroacetic acid, malic acid, maleic acid, malonic acid, fumaric acid, succinic acid, succinic acid monoamide, glutamic acid, tartaric acid, oxalic acid, citric acid, glycolic acid, glucuronic acid, ascorbic acid, benzoic acid, phthalic acid, salicylic acid, anthranilic acid, dichloroacetic acid, aminooxyaceitc acid, benzenesulfonic acid, p-toluenesulfonic acid or methanesulfonic acid. The inorganic acid may be, for example, hydrochloric acid, hydrobromic acid, sulfuric acid, phosphoric acid, nitric acid, carbonic acid or boric acid. The acid addition salt is preferably a hydrochloride or acetate, and more preferably a hydrochloride.

In addition, a form that can be a salt may be a GABA salt, a gabapentin salt, a pregabalin salt, a nicotinate salt, an adipate salt, a hemimalonate salt, a cysteine salt, an acetylcysteine salt, a methionine salt, an arginine salt, a lysine salt, an ornithine salt or an aspartate salt.

In addition, the pharmaceutical composition of the present invention may further include a pharmaceutically acceptable carrier. The pharmaceutically acceptable carrier may further include, for example, a carrier for oral administration or a carrier for parenteral administration. The carrier for oral administration may be lactose, starch, a cellulose derivative, magnesium stearate, or stearic acid. The carrier for parenteral administration may be water, a suitable oil, physiological saline, aqueous glucose and glycol. In addition, a stabilizer and a preservative may be included. A suitable stabilizer is sodium bisulfite, sodium sulfite, or an antioxidant such as ascorbic acid. A suitable preservative is benzalkonium chloride, methyl-or propyl-paraben, or chlorobutanol. For other pharmaceutically acceptable carriers, reference may be made to those disclosed in the following document (Remington's Pharmaceutical Sciences, 19th ed., Mack Publishing Company, Easton, PA, 1995).

The pharmaceutical composition of the present invention may be administered into mammals including a human by any method. For example, the pharmaceutical composition of the present invention may be administered orally or parenterally, and a parenteral administration method may be, but is not limited to, intravenous, intramuscular, intraarterial, intramedullary, intrathecal, intracardiac, transdermal, subcutaneous, intraperitoneal, intranasal, enteral, local, sublingual or rectal administration.

The pharmaceutical composition of the present invention may be formulated as a preparation for oral or parenteral administration according to an administration route described above. For the formulation of the composition, the oral formulation may be prepared using one or more buffers (e.g., saline or PBS), an antioxidant, an antibacterial agent, a chelating agent (e.g., EDTA or glutathione), a filler, an expander, a binder, an adjuvant (e.g., aluminum hydroxide), a suspending agent, a thickening agent, a wetting agent, a disintegrant, a surfactant, a diluent or an excipient.

Solid preparations for oral administration may include tablets, pills, powders, gels, slurries, suspension, and capsules, and these solid preparations may be prepared by mixing the pharmaceutical composition of the present invention with at least one or more excipients, for example, starch (including corn starch, wheat starch, rice starch, potato starch, etc.), calcium carbonate, sucrose, lactose, dextrose, sorbitol, mannitol, xylitol, erythritol, maltitol, cellulose, methyl cellulose, sodium carboxymethylcellulose, hydroxyproxymethyl-cellulose or gelatin. For example, tablets or sugar-coated tablets may be obtained by combining an active ingredient with solid excipients, grinding the mixture, adding a suitable adjuvant and processing the resulting product into a granular mixture.

Other than simple excipients, lubricants such as magnesium stearate, talc, etc. are also used. As a liquid preparation for oral administration, a suspending agent, a liquid for internal use, an emulsion or a syrup is used, and other than a commonly used simple diluent such as water or a liquid paraffin, various excipients, for example, a wetting agent, a sweetening agent, a fragrance and a preservative may be included.

In addition, in some cases, crosslinked polyvinylpyrrolidone, agar, alginic acid or sodium alginate may be added as a disintegrant, and an anticoagulant, an aromatic, an emulsifier, a solubilizer, a dispersant, a flavoring agent, an antioxidant, a packaging agent, a pigment and a preservative may be further included.

For parenteral administration, the pharmaceutical composition of the present invention may be formulated in the form of an injection, an agent for transdermal administration and a nasal inhalant together with a suitable parenteral carrier by a method known in the art. The injection needs to be sterilized and protected from contamination by microorganisms such as bacteria and fungi. In the case of injection, examples of suitable carriers may be, but are not limited to, water, ethanol, polyols (e.g., glycerol, propylene glycol and liquid polyethylene glycol), a mixture thereof and/or a solvent or dispersion medium containing vegetable oil. More preferably, as a suitable carrier, an isotonic solution such as Hank's solution, Ringer's solution, triethanol amine-containing phosphate buffered saline (PBS) or injectable sterile water, 10% ethanol, 40% propylene glycol and 5% dextrose may be used. To protect the injection from microbial contamination, various antibacterial agents and antifungal agents such as paraben, chlorobutanol, phenol, sorbic acid and thimerosal may be further included. In addition, the injection may further include, in most cases, an isotonic agent such as a sugar or sodium chloride.

The agent for transdermal administration is prepared in an ointment, a cream, a lotion, a gel, a liquid for external use, a pasta, a liniment or an aerosol. The "transdermal administration" means that an effective amount of active ingredient contained in a pharmaceutical composition is delivered into the skin by topically administering the pharmaceutical composition to the skin.

In the case of an agent administered by inhalation, the compound used according to the present invention may be conveniently delivered in the form of an aerosol spray from a pressurized pack or nebulizer, using a suitable propellant, for example, dichlorofluoromethane, trichlorofluoromethane, dichlorotetrafluoroethane, carbon dioxide or another suitable gas. In the case of a pressurized aerosol, a dosage unit may be determined by providing a valve for delivering a measured amount. For example, a gelatin capsule and a cartridge used in an inhaler or insufflator may be formulated to contain a powder mixture of a compound, and a suitable powder base such as lactose or starch. Formulations for parenteral administration are disclosed in prescriptions generally known in all of pharmaceutical chemistry (Remington's Pharmaceutical Science, 15th Edition, 1975. Mack Publishing Company, Easton, Pennsylvania 18042, Chapter 87: Blaug, Seymour).

The pharmaceutical composition of the present invention may provide a preferable effect of preventing, improving or treating a bone loss-related disease when CHP or a salt thereof and PTH are contained at effective amounts. The term "effective amount" used herein refers to an amount that shows a greater response than a negative control, and preferably, an amount sufficient for preventing, improving or treating a bone loss-related disease. The CHP or a salt thereof and PTH may be contained at 0.01 to 99.9% in the pharmaceutical composition of the present invention, and the remainder may be occupied by a pharmaceutically acceptable carrier. An effective amount of the CHP or a salt thereof and PTH included in the pharmaceutical composition of the present invention may vary according to a commercialized form of the composition.

The total effective amount of the pharmaceutical composition of the present invention may be administered to a patient in a single dose, or administered by a fractionated treatment protocol for administration for a long time in multiple doses. An effective amount of the active ingredient in the pharmaceutical composition of the present invention may vary according to the degree of disease. For example, the CHP of the pharmaceutical composition of the present invention may be administered daily once or several times to be administered at preferably 0.001 to 100 mg, and more preferably 0.01 to 10 mg per kg of body weight. However, since an effective dosage of the CHP or a salt thereof and PTH for a patient may be determined by considering various factors such as a patient's age, body weight, health condition and gender, the severity of a disease, a diet and an excretion rate, as well as the administration route of the pharmaceutical composition and the number of treatments by one of ordinary skill in the art, a suitably effective dosage of the CHP or a salt thereof and PTH may be determined according to a specific use for preventing, treating or improving a bone loss-related disease. The pharmaceutical composition according to the present invention is not particularly limited in its formulation, administration route and administration method as long as the effect of the present invention is exhibited.

The pharmaceutical composition for preventing or treating a bone loss-related disease according to the present invention may be used independently, or in combination with surgery, radiotherapy, hormonal therapy, chemotherapy or methods using a biological reaction modifier.

The pharmaceutical composition for preventing or treating a bone loss-related disease according to the present invention may also be provided in a formulation for external use, including CHP or a salt thereof and PTH. In this aspect, the composition of the present invention may be a quasi-drug composition for preventing or improving a bone loss-related disease and a quasi-drug including the composition.

Ther preparation for external use may be directly applied to the skin or mouth. When the pharmaceutical composition for preventing or treating a bone loss-related disease according to the present invention is used in the preparation for external use, an adjuvant commonly used in the field of dermatology, which is the same as any other ingredient conventionally used for a preparation for topical use, such as a lipid material, an organic solvent, a solubilizer, a thickening agent and a gelling agent, an emollient, an antioxidant, a suspending agent, a stabilizer, a foaming agent, a fragrance, a surfactant, water, an ionic emulsifier, a non-ionic emulsifier, a filler, a metal sequestering agent, a chelating agent, a preservative, a vitamin, a blocking agent, a wetting agent, an essential oil, a dye, a pigment, a hydrophilic activator, a lipophilic activator or a lipid vesicle, may be further contained. In addition, the ingredients may be introduced at an amount generally used in the field of dermatology.

When the composition of the present invention is provided as a preparation for external use, it may be prepared in the form of a liquid, an ointment, a patch, a gel, a cream or a spray, but the present invention is not limited thereto. According to one embodiment of the present invention, the quasi-drug of the present invention may include oral care products including a tooth paste, a mouthwash and a mouth spray, an ointment, a mask, a poultice, a plaster, and a patch.

When the CHP of the present invention is treated, osteoblast differentiation is promoted, and therefore, when the active ingredient is applied to an oral care product, osteoblast differentiation is promoted and thus there is an effect of preventing or improving an alveolar bone-related disease. Accordingly, the composition for a preparation for external use may be a composition for oral care to prevent or improve bone loss.

When the composition of the present invention is used as the composition for a preparation for external use, the CHP or a salt thereof and PTH may be suitably used alone or in combination of other ingredients for a quasi-drug by a conventional method. The mixing amount of the active ingredient may be suitably determined according to the purpose of use (prevention, health or therapeutic treatment).

The contents of the pharmaceutical composition and health functional food composition of the present invention may be applied mutatis mutandis to the quasi-drug composition and quasi-drug of the present invention.

In the health functional food composition of the present invention, as the "sitologically acceptable salt," an acid addition salt formed by a sitologically acceptable free-acid or a metal salt formed by a base is useful. In one example, as free acids, an inorganic acid and an organic acid may be used. The inorganic acid may be hydrochloric acid, sulfuric acid, hydrobromic acid, sulfurous acid or phosphoric acid, and the organic acid may be citric acid, acetic acid, maleic acid, fumaric acid, gluconic acid, or methanesulfonic acid. In addition, the metal salt may be an alkaline metal salt, an alkaline earth metal salt, or a sodium, potassium or calcium salt. However, the present inventio is not limited thereto.

The term "health functional food" used herein includes both "functional food" and "health food."

The term "functional food" used herein is a term the same as a food for special health use (FoSHU), and refers to a food with high medical efficacy, processed to efficiently exhibit bioregulatory functions in addition to nutritional supply.

The term "health food" refers to a food with an effect of active health maintenance or a promotion effect compared to general foods, and the health supplement food refers to food for dietary supplements. In some cases, the terms such as functional food, health food, and health supplementary food are used interchangeably. The food may be prepared in various formulations such as a tablet, a capsule, a powder, a granule, a liquid, and a pill, to obtain an efficient effect in improving or recovering a bone loss-related disease.

As a specific example of such functional food, by using the composition, a processed food with an improved storage property as well as for modifying characteristics of agricultural, livestock or aquatic products may be prepared.

The health functional food composition of the present invention may be prepared in the form of a nutritional supplement, a food additive and feed, which is for animals such as a human or livestock.

These types of food composition may be prepared in various forms according to conventional methods known in the art. Common foods may be prepared by adding CHP or a salt thereof and PTH to beverages (including alcoholic beverages), fruits and processed foods thereof (e.g., canned fruit, preserved fruit, jam, marmalade, etc.), fish, meat and a processed food thereof (e.g., ham, sausage corn beef, etc.), breads and noodles (e.g., udon, soba, ramen, spaghetti, macaroni, etc.), fruit juice, various drinks, cookies, taffy, dairy products (e.g., butter, cheese, etc.), edible vegetable oil, margarine, vegetable protein, retort food, frozen food, various seasonings (e.g., a bean paste, a soy sauce, a source, etc.), but the present invention is not limited thereto.

In addition, the nutritional supplement may be prepared by adding CHP or a salt thereof and PTH to a capsule, a tablet or a pill, but the present invention is not limited thereto.

In addition, as the health functional food, the CHP or a salt thereof and PTH may be prepared in the form of a tea, juice, and liquefied, granulated, encapsulated and powdered to drink (as a health drink), but the present invention is not limited thereto. In addition, the CHP or a salt thereof and PTH may be used by being prepared in the form of a powder or concentrate as a food additive. In addition, the CHP or a salt thereof and PTH may be mixed with an active ingredient known to have an effect in preventing or improving a bone loss-related disease, thereby obtaining a composition.

When the food composition is used as a health drink composition, the health drink composition may contain several flavoring agents or natural carbohydrates as additional ingredients, like common drinks. Examples of the above-mentioned natural carbohydrates include conventional sugars, for example, monosaccharides such as glucose and fructose; disaccharides such as maltose and sucrose; and polysaccharides such as dextrin and cyclodextrin, and sugar alcohols such as xylitol, sorbitol and erythritol. As the sweeteners, natural sweeteners such as thaumatin and stevia extract, and synthetic sweeteners such as saccharin and aspartame may be used. The proportion of the natural carbohydrate may be generally about 0.01 to 0.04 g, and preferably, about 0.02 to 0.03 g per 100 mL of the composition of the present invention.

The CHP or a salt thereof and PTH may be contained as an active ingredient of a food composition for preventing or improving a bone loss-related disease, and the amount thereof is an amount effective to obtain the preventive or improving effect, and is preferably, for example, 0.01 to 100 wt% with respect to the total weight of the entire composition, but the present invention is not particularly limited thereto. The food composition of the present invention may be prepared by mixing the CHP or a salt thereof and PTH with another active ingredient known to have an effect of preventing or improving a bone loss-related disease.

In addition, the health functional food of the present invention may contain various nutrients, vitamins, electrolytes, flavoring agents, pectic acid and a salt thereof, alginic acid and a salt thereof, organic acids, protective colloidal thickening agents, pH adjustors, stabilizers, preservatives, glycerin, alcohols, or carbonizing agents. Other than these, the composition of the present invention may contain fruit pulp for producing natural fruit juices, fruit drinks and vegetable drinks. These ingredients may be used independently or in combination. The proportion of such an additive, while not particularly important, is generally selected from 0.01 to 0.1 parts by weight per 100 parts by weight of the composition of the present invention.

The present invention also provides a method of preventing or treating a bone loss-related disease, which includes administering an effective amount of a composition including CHP or a pharmaceutically acceptable salt thereof and PTH to a subject in need thereof.

The present invention also provides a method of improving a therapeutic effect of PTH on a bone loss-related disease, which includes administering CHP or a pharmaceutically acceptable salt thereof and PTH into a subject in need thereof.

The term "subject" used herein includes any kinds of animals (e.g., humans, horses, pigs, rabbits, dogs, sheep, goats, non-human primates, cattle, cats, guinea pigs and rodents), but the present invention is not limited thereto. This term does not infer a specific age or gender. Thus, the subject is intended to include an adult, a neonatal subject regardless of a female or male, as well as a fetus. A patient refers to a subject with a disease or disorder. The term "patient" includes a human and a veterinary subject.

In the method of the present invention, the descriptions of the effects of CHP and PTH and an administration route thereof, the number of administrations, and a dosage are the same as above, and will be omitted.

In the method of the present invention, the CHP or a pharmaceutically acceptable salt; and PTH may be administered simultaneously, separately or sequentially. For example, the CHP or a pharmaceutically acceptable salt and PTH may be co-formulated to be simultaneously administered as a unit dose preparation, or simultaneously or sequentially administered as separate preparations.

When sequentially administered, each active ingredient may be administered at regular intervals, and the order of administrations may be determined by a physician or one of ordinary skill in the art.

The present invention also provides a use of a composition including CHP or a pharmaceutically acceptable salt and PTH in the preparation of a drug for preventing or treating a bone loss-related disease.

The present invention also provides a use of a composition including CHP or a pharmaceutically acceptable salt thereof in the preparation of a drug for improving a preventive or therapeutic effect of PTH on a bone loss-related disease.

Hereinafter, the present invention will be described in further detail with reference to examples. The present invention may have various modifications and various examples, and thus specific examples are illustrated in the drawings and described in detail in the detailed description.

### [Examples]

### [Preparation Example 1]

CHP used in the following examples was purchased from Bachem, and human parathyroid hormone 1-34 (hPTH₁₋₃₄) was purchased from Tocris. For MC3T3-E1 preosteoblast culture, MEMα (Gibco), an ascorbic acid-free medium and FBS (Hyclone) were used, and β-glycerophosphate and ascorbic acid, which are used in differentiation, were purchased from Sigma-Aldrich. For cell lysis, a RIPA lysis buffer (Thermo) was used. For measurement of alkaline phosphatase (ALP) activity, a pNPP substrate solution (Sigma-Aldrich) was used, and for protein quantification, a BCA quantification kit (Thermo) was used. For RNA extraction from cells, NucleoZOL (MACHEREY-NAGEL) was purchased, and for cDNA synthesis and real-time PCR, an iScript cDNA synthesis kit (Bio-Rad) and iQ SYBR Green Supermix were purchased.

### [Preparation Example 2]

For culture of calvarial primary osteoblasts, a one-day-old C57BL/6 pup was purchased from Koatech. Collagenase and dispase for cell isolation were purchased from Gibco and Roche, respectively.

### [Preparation Example 3]

Primers for real-time PCR are base sequences shown in Table 1, which were synthesized by Bioneer.

**[Table 1]**

| **Name of gene** | **Forward primer** | **SEQ ID NO:** | **Reverse primer** | **SEQ ID NO:** |
|---|---|---|---|---|
| ALP | 5'-agatggcctggatctcatca-3' | 3 | 5'-cagtgcggttccagacatag-3' | 4 |
| BMP2 | 5'-tggaagtggcccatttagag-3' | 5 | 5'-tgacgcttttctcgtttgtg-3' | 6 |
| Osteocalcin | 5'-ctgctcactctgctgacc-3' | 7 | 5'-ttgtcagactcagggccg-3' | 8 |
| Osterix | 5'-gaagtccaatggggatctga-3' | 9 | 5'-agaatccctttccctctcca-3' | 10 |
| Runx2 | 5'-aagtgcggtgcaaactttctc-3' | 11 | 5'-tgcttgcagccttaaatattcc-3' | 12 |
| Colla1 | 5'-ctaacgtggttcgtgaccg-3' | 13 | 5'-gctgcggatgttctcaatct-3' | 14 |
| OPG | 5'-ggaatagatgtcaccctgtgt-3' | 15 | 5'-aaaacactcagccaatttggtat-3' | 16 |
| β-actin | 5'-gggaaggtgacagcattg-3' | 17 | 5'-atgaagtattaaggcggaagatt-3' | 18 |

### [Example 1]

Measurement of efficacy of promotion of MC3T3-E1 osteoblast differentiation and bone formation according to combined administration of CHP and hPTH₁₋₃₄

### 1-1. Conditions for MC3T3-E1 osteoblast culture

Using a MEMα medium containing 10% FBS and 1% penicillin/streptomycin and an ascorbic acid-free medium, MC3T3-E1 cells were incubated in an incubator with 5% CO₂ at 37 °C. For differentiation into osteoblasts, when the cells were grown to confluency, 10 mM β-glycerophosphate and 50 µg/µL ascorbic acid were added to the composition of the above basal medium composition.

### 1-2. Promotion of induction of MC3T3-E1 osteoblast differentiation by single and combined treatment with CHP and hPTH₁₋₃₄

2.5x10⁵ MC3T3-E1 cells were seeded at 1 mL in 12-well plates, and grown to confluency for 48 hours. For differentiation to osteoblasts, the medium was replaced with a differentiation medium, and treated with CHP and hPTH₁₋₃₄ at corresponding concentrations for each group as shown in Table 2. Here, for an undifferentiated group, which is a negative control for differentiation, a basal medium, not a differentiation medium, was used. The medium was removed from all groups after six hours of treatment, and replaced with a differentiation medium after washing with PBS once. Each concentration of CHP and hPTH₁₋₃₄ was treated while replacing the medium every 72 hours, and cultured for a total of 14 days.

**[Table 2]**

| Group | 10mM | hPTH₁₋₃₄ | CHP |
|---|---|---|---|
| | β-glycerophosphate | (washing after 6 hrs) | (washing after 6 hrs) |
| | 50 µg/µL ascorbic acid | | |
| Undifferentiated group | - | - | - |
| Negative control | + | - | - |
| CHP only-treated group 1 (1 mM) | + | - | 1 mM |
| hPTH₁₋₃₄ only-treated group 1 (1 nM) | + | 1 nM | - |
| hPTH₁₋₃₄ only-treated group 2 (2 nM) | + | 2 nM | - |
| Combined treatment group 1 | + | 1 nM | 1 mM |
| (PTH 1 nM - CHP 1 mM) | | | |
| Combined treatment group 2 | + | 2 nM | 1 mM |
| (PTH 2 nM - CHP 1 mM) | | | |

### 1-3. Measurement of ALP activity

On day 14 of differentiation, the medium was removed, the cells were washed with PBS once, 100 µL of a RIPA lysis buffer was added to each well, followed by detaching the cells using a cell scraper. The cells were transferred into a 1.5 mL tube using a pipette, and disrupted using a sonicator (amplitude of 10, 0.5 sec pulse, 10 times). After centrifugation at 4 °C and 15,000 rpm for 10 minutes, only a supernatant was isolated. 10 µL of the isolated lysate sample was added first to a 96-well plate, and 200 µL of a pNPP substrate solution was added to allow a reaction at room temperature for 1 hour, followed by measuring absorbance at 405 nm.

To calibrate the ALP activity level to the protein concentrations of the lysates, each lysate was quantified by a BCA quantification method, and thus the ALP activity absorbance value at 405 nm was divided by a total amount of proteins added [Abs. OD₄₀₅ₙₘ/µg of analyzed].

For statistical significance, one-way ANOVA statistical analysis was used, and the Tukey post hoc test was used to compare significance with a control (*p<0.05).

As a result of the experiment, as shown in FIGS. 1A and 1B, it was confirmed that the ALP activity in a group treated with hPTH₁₋₃₄ and CHP in combination was higher than that of a hPTH₁₋₃₄ only-treated group. This result can be interpreted as that the action of promoting osteoblasts differentiation and bone formation by hPTH₁₋₃₄ is more increased by combined administration of CHP. According to this, it was confirmed that the combined administration of hPTH₁₋₃₄ and CHP exhibits a synergistic effect on bone health and osteoporosis treatment.

### [Example 2]

Measurement of expression of genes associated with primary osteoblast differentiation and bone formation according to combined administration of CHP and hPTH₁₋₃₄

### 2-1. Primary osteoblast culture

Only the calvaria was isolated from 1-day-old C57BL/6 pup. The isolated calvaria was added to a digestion medium (0.1% collagenase and 0.2% dispaseadded MEMα), shaken at 37 °C for digestion five times for 15 minutes each. The first digested solution was discarded, and the digested solutions from the second to fifth times were collected. The resulting solutions were centrifuged at 1,600 rpm for 5 minutes, and the resulting cells were suspended in a culture medium (10% FBS-added MEMα), and then debris were removed through a 0.2 µm strainer. The cells obtained from the culture medium were resuspended, and incubated at 37 °C in a 5% CO₂ incubator for 3 days. Afterward, for differentiation to osteoblasts, when the cells were grown to confluency, 10 mM β-glycerophosphate and 50 µg/ µL ascorbic acid were added to the culture medium composition and used.

### 2-2. Promotion of induction of primary osteoblast differentiation by single treatment and combined treatment of CHP and hPTH₁₋₃₄

1x10⁵ primary osteoblasts were seeded at 1 mL in 12-well plates, and incubated for 48 hours. For differentiation to osteoblasts, the medium was replaced with a differentiation medium, and CHP and hPTH₁₋₃₄ were treated at corresponding concentrations for each group as shown in Table 3. Here, for an undifferentiated group, which is a negative control for differentiation, a basal culture medium, not a differentiation medium, was used. For all groups, the medium was removed after six hours of treatment, and the cells were washed with PBS once, followed by replacing with a differentiation medium. While replacing the medium every 48 hours, different concentrations of CHP and hPTH₁₋₃₄ were treated, and incubated for a total of six days.

**[Table 3]**

| Group | 10mM | hPTH₁₋₃₄ | CHP |
|---|---|---|---|
| | B-glycerophosphate | (washing after 6 hrs) | (washing after 6 hrs) |
| | 50 µg/µL ascorbic acid | | |
| Undifferentiated group | - | - | - |
| Negative control | + | - | - |
| CHP only-treated group 1 (1 mM) | + | - | 1mM |
| hPTH₁₋₃₄ only-treated group (1 nM) | + | 1nM | - |
| Combined treatment group | + | 1nM | 1mM |
| (PTH 1 nM - CHP 1 mM) | | | |

### 2-3. RNA extraction in cells

On day 6 of differentiation, the medium was removed and washed with PBS once, and then 500 µL of NucleoZOL was added to lyze the cells. Subsequently, RNA was extracted according to the total RNA isolation protocol of NucleoZOL, and cDNA was synthesized from 1 µg of RNA by Reverse Transcription Polymerase Chain Reaction (PCR) using an iScript cDNA synthesis kit.

### 2-4. Analysis of expression of genes associated with osteoblast differentiation and bone formation

The synthesized cDNA was analyzed with a forward/reverse primer set suitable for each gene through real-time PCR using iQ SYBR Green Supermix. The expression level of each gene was corrected by division by the expression level of a housekeeping gene, β-actin. Statistical significance was determined by one-way ANOVA, and the Tukey post hoc test was used to compare the significance with a control (*p<0.05, **p<0.01).

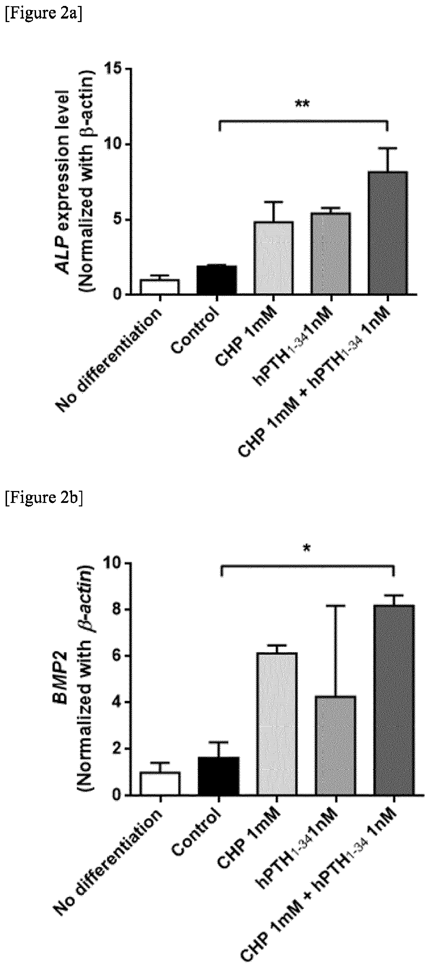

As a result of the experiment, as shown in FIG. 2A, it was confirmed that, in the group co-treated with hPTH₁₋₃₄ and CHP, the expression of the alkaline phosphatase (ALP) gene, which is a representative marker for osteoblast differentiation, is significantly increased compared to the control, and also significantly increased compared to both CHP- and hPTH₁₋₃₄ only-treated groups. Therefore, it can be seen that by the combined administration of hPTH₁₋₃₄ and CHP, the action of promoting primary osteoblast differentiation and bone formation was dramatically increased.

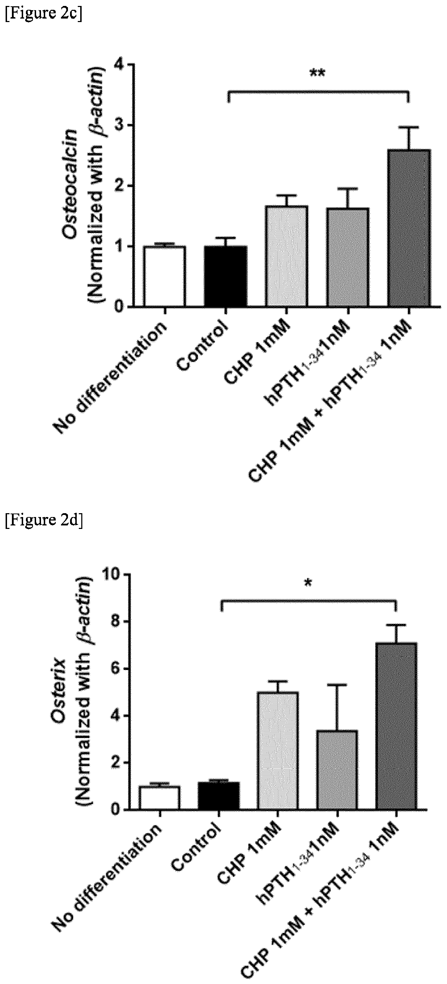

As shown in FIGS. 2B and 2C, it can be seen that BMP2 and Osteocalcin genes secreted by osteoblast differentiation were significantly increased in the group co-treated with hPTH₁₋₃₄ and CHP. Therefore, it can be seen that by the combined administration of hPTH₁₋₃₄ and CHP, primary osteoblast differentiation was dramatically promoted to a significant level compared to the control.

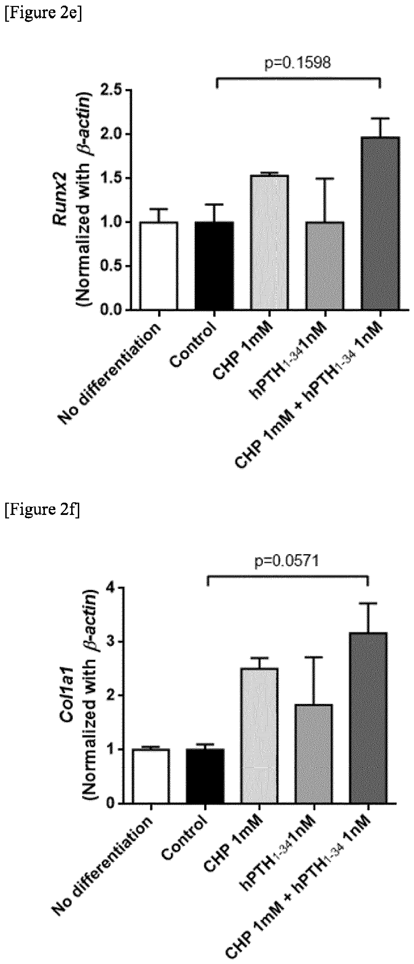

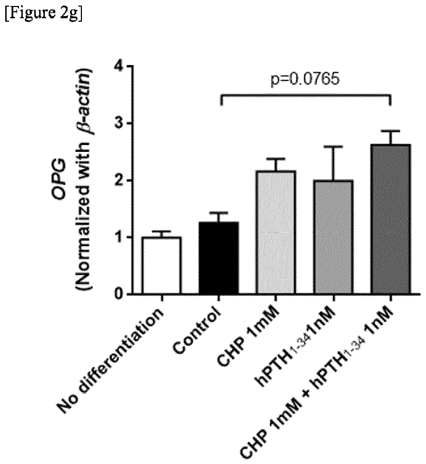

As shown in FIGS. 2D and 2E, it was confirmed that the expression of Osterix and Runx2 genes, which are transcription factors involved in osteoblast differentiation and bone formation, was significantly increased in the group co-treated with hPTH₁₋₃₄ and CHP, compared to the control. In addition, as shown in FIGS. 2F and 2G, the expression of Collagen lal (Collal) and Osteoprotegerin (OPG) genes increased in response to signaling of osteoblast differentiation and bone formation was also likely to increase close to a significant level in the group co-treated with hPTH₁₋₃₄ and CHP, compared to the control.

From the above results, it was confirmed that the combined administration of hPTH₁₋₃₄ and CHP results in a synergistic effect on primary osteoblast differentiation and bone formation, greater than the predicted effect from each single administration. Therefore, it can be seen that the combined administration of hPTH₁₋₃₄ and CHP has the unpredictably excellent therapeutic efficacy on bone health and treatment of bone loss-related diseases including osteoporosis.

## Claims

1. A composition for use in preventing, improving or treating a bone loss-related disease, comprising cyclo(his-pro) (CHP) or a pharmaceutically acceptable salt thereof; and parathyroid hormone (PTH).

2. The composition for use as claimed in claim 1, wherein PTH is PTH₁₋₃₄.

3. The composition for use as claimed in claim 1, wherein the bone loss-related disease is any one or more selected from the group consisting of osteoporosis, Paget's disease, alveolar bone loss, osteomalacia, and renal osteodystrophy.

4. The composition for use as claimed in claim 3, wherein the osteoporosis is caused by a decrease in female hormone levels, or the destruction or inhibition of the activity of osteoblasts.

5. A composition for use in improving an effect of parathyroid hormone (PTH) for preventing, improving or treating a bone loss-related disease, comprising cyclo(his-pro) (CHP) or a pharmaceutically acceptable salt thereof.

6. The composition for use as claimed in claim 5, wherein the PTH is PTH₁₋₃₄.

7. The composition for use as claimed in claim 5, wherein the bone loss-related disease is any one or more selected from the group consisting of osteoporosis, Paget's disease, alveolar bone loss, osteomalacia, and renal osteodystrophy.

8. The composition for use as claimed in claim 7, wherein the osteoporosis is caused by a decrease in female hormone levels, or the destruction or inhibition of the activity of osteoblasts.

## Patentansprüche

1. Zusammensetzung zur Verwendung bei der Vorbeugung, Verbesserung oder Behandlung einer mit Knochenschwund verbundenen Erkrankung, umfassend Cyclo(His-Pro) (CHP) oder ein pharmazeutisch verträgliches Salz davon; und Parathyroidhormon (PTH).

2. Zusammensetzung zur Verwendung nach Anspruch 1, wobei PTH PTH₁₋₃₄ ist.

3. Zusammensetzung zur Verwendung nach Anspruch 1, wobei die mit Knochenschwund verbundene Erkrankung eine oder mehrere, ausgewählt aus der Gruppe bestehend aus Osteoporose, Paget-Krankheit, alveolärem Knochenschwund, Osteomalazie und renaler Osteodystrophie, ist.

4. Zusammensetzung zur Verwendung nach Anspruch 3, wobei die Osteoporose durch eine Abnahme der Spiegel der weiblichen Hormone oder den Abbau oder die Hemmung der Aktivität von Osteoblasten verursacht wird.

5. Zusammensetzung zur Verwendung bei der Verbesserung einer Wirkung von Parathyroidhormon (PTH) zur Vorbeugung, Verbesserung oder Behandlung einer mit Knochenschwund verbundenen Erkrankung, umfassend Cyclo(His-Pro) (CHP) oder ein pharmazeutisch verträgliches Salz davon.

6. Zusammensetzung zur Verwendung nach Anspruch 5, wobei das PTH PTH₁₋₃₄ ist.

7. Zusammensetzung zur Verwendung nach Anspruch 5, wobei die mit Knochenschwund verbundene Erkrankung eine oder mehrere, ausgewählt aus der Gruppe bestehend aus Osteoporose, Paget-Krankheit, alveolärem Knochenschwund, Osteomalazie und renaler Osteodystrophie, ist.

8. Zusammensetzung zur Verwendung nach Anspruch 7, wobei die Osteoporose durch eine Abnahme der Spiegel der weiblichen Hormone oder den Abbau oder die Hemmung der Aktivität von Osteoblasten verursacht wird.

## Revendications

1. Composition pour une utilisation dans la prévention, l'amélioration ou le traitement d'une maladie reliée à la perte osseuse, comprenant du cyclo(his-pro) (CHP) ou un de ses sels pharmaceutiquement acceptable ; et de l'hormone parathyroïde (PTH).

2. Composition pour une utilisation selon la revendication 1, dans laquelle la PTH est la PTH₁₋₃₄.

3. Composition pour une utilisation selon la revendication 1, dans laquelle la maladie reliée à la perte osseuse est une ou plusieurs maladies quelconques sélectionnées dans le groupe constitué de l'ostéoporose, la maladie de Paget, la perte osseuse alvéolaire, l'ostéomalacie et l'ostéodystrophie rénale.

4. Composition pour une utilisation selon la revendication 3, dans laquelle l'ostéoporose est causée par une diminution des niveaux d'hormones féminines, ou la destruction ou l'inhibition de l'activité des ostéoblastes.

5. Composition pour une utilisation dans l'amélioration de l'effet de l'hormone parathyroïde (PTH) pour la prévention, l'amélioration ou le traitement d'une maladie reliée à la perte osseuse, comprenant du cyclo(his-pro) (CHP) ou un de ses sels pharmaceutiquement acceptable.

6. Composition pour une utilisation selon la revendication 5, dans laquelle la PTH est la PTH₁₋₃₄.

7. Composition pour une utilisation selon la revendication 5, dans laquelle la maladie reliée à la perte osseuse est une ou plusieurs maladies quelconques sélectionnées dans le groupe constitué de l'ostéoporose, la maladie de Paget, la perte osseuse alvéolaire, l'ostéomalacie et l'ostéodystrophie rénale.

8. Composition pour une utilisation selon la revendication 7, dans laquelle l'ostéoporose est causée par une diminution des niveaux d'hormones féminines, ou la destruction ou l'inhibition de l'activité des ostéoblastes.
